# EUROPEAN PATENT APPLICATION

(11) **EP 0 817 208 A1**
(43) Date of publication of application: **07.01.1998**
(21) Application number: 97110597.8
(22) Date of filing: 27.06.1997
(51) Int. Cl.: G21K 1/04

(54) **Apparatus and method for adjusting radiation in a radiation-emitting device**

(30) Priority: 28.06.1996 US 671899
(71) Applicant: SIEMENS MEDICAL SYSTEMS, INC., Iselin, New Jersey 08830 (US)
(72) Inventor: Hughes, John H., Martinez, CA. 94553 (US); Hernandez, Francisco M., Concord, CA. 94518 (US)
(74) Representative: Blumbach, Kramer & Partner

(57) **Abstract**

In a radiation emitting device, particularly in a radiation treatment device (2), the actual radiation delivered to an object (13) via a radiation beam (1) is adjusted dependent on various parameters. Jaws (100, 102, 104, 106) are arranged between a radiation source and an object (13) to provide opening (110). The radiation delivered to the object is a function of (i) opening (110) in jaws (100, 102, 104, 106), (ii) the position of the radiation source, and (iii) the output radiation from a radiation source. Calculations are performed to provide beam patterns (202-206). These beam patterns (202-206) provide the parameters needed for the desired radiation treatment.

## Description

### BACKGROUND OF THE INVENTION

### Field of the Invention

The invention relates to a radiation emitting device, and particularly to a system and a method for adjusting the radiation delivered to an object in a radiation treatment device.

### Description of the Related Art

Radiation-emitting devices are generally known and used, for instance, as radiation therapy devices for the treatment of patients. A radiation therapy device usually comprises a gantry which can be swiveled around a horizontal axis of rotation in the course of a therapeutic treatment. A linear accelerator is located in the gantry for generating a high-energy radiation beam for therapy. This high energy radiation beam can be an electron radiation or photon (X-ray) beam. During treatment, this radiation beam is trained on a zone of a patient lying in the isocenter of the gantry rotation.

To control the radiation emitted toward an object, a beam-shielding device such as a plate arrangement and/or collimator is usually provided in the trajectory of the radiation beam between the radiation source and the object. This beam-shielding device defines a field on the object to which a prescribed amount of radiation is to be delivered.

The radiation delivered to an object may be analyzed into primary and scattered components. The primary radiation is made up of the initial or original photons emitted from the radiation source, and the scattered radiation is the result of the photons scattered by the plate arrangement itself. The beam's radiation output in free space increases because of the increased plate/collimator scatter, which is added to the primary beam. In other words, a point in the field is subjected not only to direct radiation (which is the primary component), but also to radiation that is scattered from the plate arrangement. The ratio of the radiation output in air with the scatterer to the radiation output without the scatterer for a reference field (for instance 10 x 10cm) is commonly called the "output factor" or the "collimator scatter factor." The concept and definition of the output factor are well understood in the art.

Thus, due to these scattered photons, the dose rate applied to the surface of the object changes dependent on the size of the opening in the plate arrangement, that is, on the field size. This means that the radiation emitted to the same spot, for instance in the center of the radiation beam onto the object, changes according to the size of the opening in the plate arrangement. When the plate arrangement shows only a small opening, then the accumulated dose at the same spot is less than the accumulated dose at the same spot when the opening is big.

Frequently, special filters or absorbing blocks are located in the trajectory of the radiation beam to modify its isodose distribution. A most commonly used filter is the wedge filter. This is a wedge-shaped absorbing block which causes a progressive decrease in the intensity across the beam, resulting in isodose curves that are modified relative to their normal positions. Such wedge filters are usually made of dense material, such as lead or steel, or other absorbing material.

The presence of a wedge filter decreases the output of the radiation-emitting device, and this decrease must be taken into account in treatment calculations. This effect is characterized as a "wedge factor." A wedge factor is defined as the ratio of doses with and without the wedge at a point in the object along the central axis of the radiation beam. The wedge factor depends on the material, size and angle of the wedge. Wedges, and in particular the wedge factor, are described in Faiz M. Khan, Ph.D, "The Physics of Radiation Therapy", Williams & Wilkins, pages 234 to 238.

The delivery of radiation by such a radiation therapy device is prescribed and approved by an oncologist. Actual operation of the radiation equipment, however, is normally done by a therapist. When the therapist administers the actual delivery of the radiation treatment as prescribed by the oncologist, the device is programmed to deliver that specific treatment. When programming the treatment, the therapist has to take into consideration the output factor and has to adjust the dose delivery based on the plate arrangement opening to achieve the prescribed radiation output on the surface of the object. This adjustment can be made according to known calculations, but the therapist normally has to do them manually, which can lead to errors. In the context of radiation therapy, a miscalculation can lead to either a dose that is too low and is ineffective, or that is too high and dangerous; a large error, for example, a misplaced decimal point, can be lethal.

U.S. Pat. No. 5,148,032 discloses a radiation treatment device in which isodose curves in the object are adjusted both by a plate arrangement, which includes at least one movable plate that is controlled during irradiation, and by varying the radiation output of the radiation beam during irradiation, so that a wide range of variations in the possible isodose curves is obtained. A wedge-shaped isodose distribution is established, for example, by moving one plate at a constant speed while simultaneously changing the radiation output of the radiation beam. In this radiation treatment device there is no physical absorbing block in the trajectory of the radiation beam, and the therapist has to take this into account.

What is needed is a method, and corresponding system, for adjusting the delivery of radiation to the object to make sure that the actually delivered radiation output is exactly the same as the desired radiation output, independent of the use of a wedge function.

### Summary of the Invention

According to the invention, radiation output delivered to an object from a radiation source is adjusted. The radiation source can be rotated to multiple available positions. First, an irradiated field on the object is defined with field parameters. A radiation beam is then generated. The radiation beam has a variable radiation output and a substantially lossless beam path from a radiation source to the object. An opening is then defined between the radiation source and the object. The opening also has multiple available parameters, and it can delimit the radiation beam to any one of the multiple available parameters. Beam patterns are then calculated based on (1) the field parameters, (2) the available positions of the radiation source, (3) the minimum and maximum amount of radiation available from the variable radiation output, and (4) the multiple available parameters for the opening. The beam patterns each include a position of the radiation source, an amount of radiation from the radiation output and parameters for the opening. The field on the object is then treated with radiation. This treatment is done while varying the position of the radiation source, the amount of radiation from the radiation output and the parameters of the opening according to the calculated beam patterns.

### Brief Description of the Drawings

Figure 1 shows a schematic diagram of a radiation treatment device including a treatment console constructed in accordance with the present invention;
Figure 2 is a block diagram illustrating portions of a processing unit, a control unit and a beam generation system in the radiation treatment device of Figure 1;
Figure 3 shows isodose curves for a conventional wedge filter built of an absorption block in the path of a radiation beam;
Figure 4 shows isodose curves for an arrangement in which a wedge filter is realized by the movement of one plate of a plate arrangement in the path of the radiation beam and by changing the radiation output of the radiation beam;
Figure 5 illustrates four jaws located in a radiation treatment device between the radiation source and the object;
Figure 6 illustrates beam patterns created by the second embodiment of the present invention;
Figure 7 provides a process flowchart for a simple software program which can be used to quickly calculate beam patterns for a desired wedge factor;
Figures 8A and 8B illustrate beam patterns created by a sweeping technique; and
Figure 9 illustrates a beam pattern created by a quadrant technique.

### Detailed Description

The invention is described below with primary reference to a system for delivering X-ray radiation to a field of a patient, and for delimiting the field using at least one movable plate or jaw in the beam path from a radiation source. The invention may be used to adjust the delivery of any type of energy, for example, electrons (instead of X-rays), to any type of object (not just a human patient), provided the amount of energy delivered to the field can be sensed or estimated.

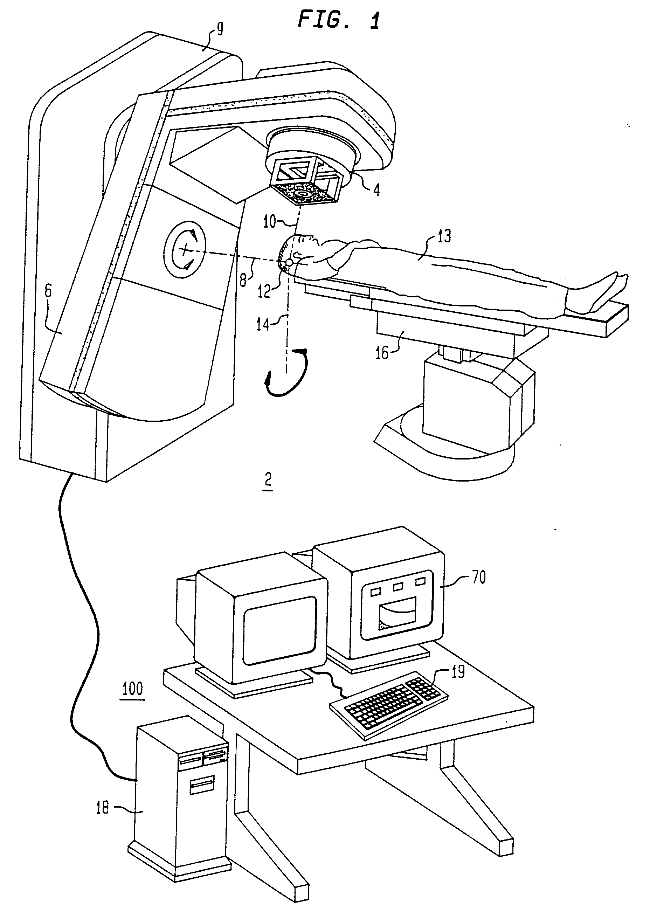

Figure 1 shows a radiation treatment device 2 of common design, in which plates 4 and a control unit in a housing 9 and a treatment unit 100 constructed in accordance with the principles of the invention are used. The radiation treatment device 2 comprises a gantry 6 which can be swiveled around a horizontal axis of rotation 8 in the course of a therapeutic treatment. Plates 4 are fastened to a projection of gantry 6. To generate the high-powered radiation required for the therapy, a linear accelerator is located in gantry 6. The axis of the radiation bundle emitted from the linear accelerator and gantry 6 is designated by 10. Electron, photon, or any other detectable radiation can be used for the therapy.

During the treatment, the radiation beam is trained on a zone 12 of an object 13 (e.g., a patient who is to be treated, and who lies at the isocenter of the gantry rotation). Rotational axis 8 of gantry 6, rotational axis 14 of the area on the object to be treated, and beam axis 10 all preferably intersect in the isocenter. The construction of such a radiation treatment device is describes in general in a brochure "Digital Systems for Radiation Oncology", Siemens Medical Laboratories, Inc. A91004-M2630-B358-01-4A00, September 1991.

The area of the patient that is irradiated is known as the field. Plates 4 are substantially impervious to the emitted radiation. They are mounted between the radiation source and the patient to delimit the field. Areas of the body, for example, healthy tissue, are therefore subjected to as little radiation as possible, and preferably to none at all. In the preferred embodiment of the invention, at least one of the plates is movable so that the distribution of radiation over the field need not be uniform (one region can be given a higher dose than another); furthermore, the gantry can preferably be rotated so as to allow different beam angles and radiation distributions without having to move the patient around. Neither or these features is necessary according to the invention: the invention may also be used with fixed-field devices (no movable plates), with constant radiation delivery rates, and with fixed-angle beams (no rotatable gantry).

Radiation treatment device 2 also includes a central treatment processing or control unit 100, which is usually located apart from radiation treatment device 2. The radiation treatment device 2 is normally located in a different room to protect the therapist from radiation. Treatment unit 100 includes output devices, such as at least one visual display unit or monitor 70, and an input device such as a keyboard 19. Data can be input also through data carriers, such as data storage devices, or a verification and recording or automatic set-up system 102, which is described below. The treatment processing unit 100 is typically operated by the therapist who administers actual delivery of a radiation treatment as prescribed by an oncologist. By utilizing the keyboard 19, or other input device, the therapist enters into a control unit 76 of the treatment unit 100 the data that defines the radiation to be delivered to the patient, for example, according to the prescription of the oncologist. The program can also be input via another input device like a data storage device, through data transmission, or using the automatic set-up system 102. On the screen of a monitor 70, various data can be displayed before and during the treatment.

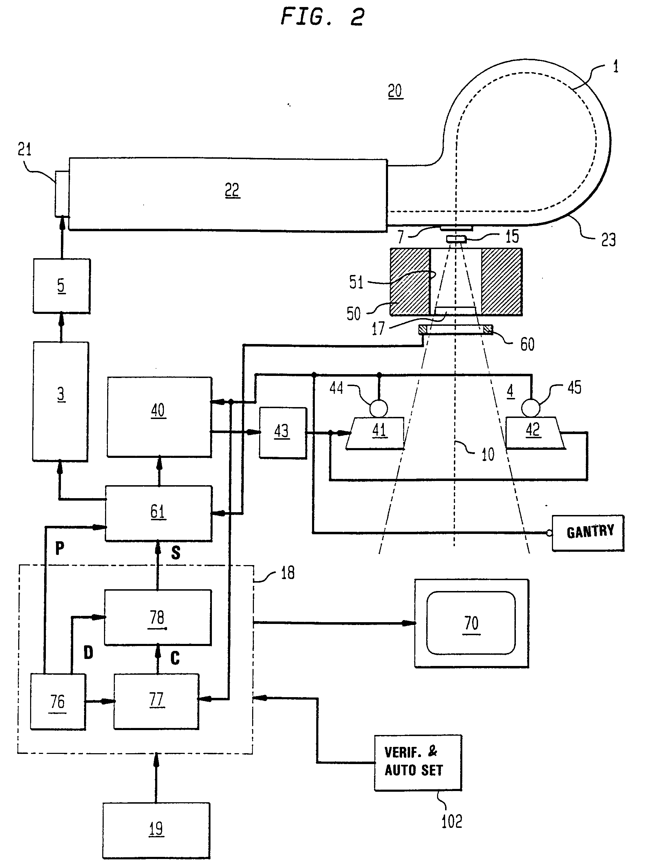

Figure 2 shows portions of an illustrative radiation treatment device 2 and portions of treatment unit 100 in more detail. An electron beam 1 is generated in an electron accelerator 20. Accelerator 20 comprises an electron gun 21, a wave guide 22 and an evacuated envelope or guide magnet 23. A trigger system 3 generates injector trigger signals and supplies them to injector 5. Based on these injector trigger signals, injector 5 generates injector pulses which are fed to electron gun 21 in accelerator 20 for generating electron beam 1. Electron beam 1 is accelerated and guided by wave guide 22. For this purpose, a high frequency (HF) source is provided which supplies radio frequency (RF) signals for the generation of an electromagnetic field supplied to wave guide 22. The electrons injected by injector 5 and emitted by electron gun 21 are accelerated by this electromagnetic field in wave guide 22 and exit at the end opposite to electron gun 21 as electron beam 1. Electron beam 1 then enters a guide magnet 23, and from there is guided through a window 7 along axis 10. After passing through a first scattering foil 15, the beam goes through a passageway 51 of a shield block 50 and encounters a second scattering foil 17. Next, it is sent through a measuring chamber 60, in which the dose is ascertained. If the scattering foils are replaced by a target, the radiation beam is an X-ray beam. Finally, aperture plate arrangement 4 is provided in the path of radiation beam 1, by which the irradiated field of the subject of investigation is determined. Aperture plate arrangement 4 includes a pair of plates 41 and 42. As is described above, this is just one example of a beam-shielding arrangement that can be used in the invention. The invention will work with others also as long as there is an aperture plate arrangement that defines an irradiated field.

Plate arrangement 4 comprises a pair of aperture plates 41 and 42 and an additional pair of aperture plates (not shown) arranged perpendicular to plates 41 and 42. To change the size of the irradiated field, each of the aperture plates can be moved with respect to axis 10 by a drive unit 43 which is indicated in Figure 2 only with respect to plate 41. Drive unit 43 comprises an electric motor which is coupled to plates 41 and 42 and which is controlled by a motor controller 40. Position sensors 44 and 45 are also coupled to plates 41 and 42, respectively, for sensing their positions. This is just one example of such a system. The invention will work with other systems also, as long as there is a beam-shielding arrangement that defines an irradiated field and as long as sensors are provided to indicate the field size. For example, the plates can be replaced with a collimator containing many (e.g., 60) radiation blocking leaves.

Motor controller 40 is coupled to a dose control unit 61 which includes a dosimetry controller and which is coupled to a central processing unit 18 for providing set values for the radiation beam for achieving given isodose curves. The output of the radiation beam is measured by a measuring chamber 60. In response to the deviation between the set values and the actual values, dose control unit 61 supplies signals to trigger system 3, which changes the pulse repetition frequency so that the deviation between the set values and the actual values of the radiation beam output is minimized.

In such a radiation treatment device, the dose absorbed by object 13 is dependent on the type of filter used for shaping the radiation beam. If a wedge filter built from absorbing material is inserted in the trajectory of the radiation beam, then the preset dose has to be increased according to the wedge factor to supply the desired dose to object 13.

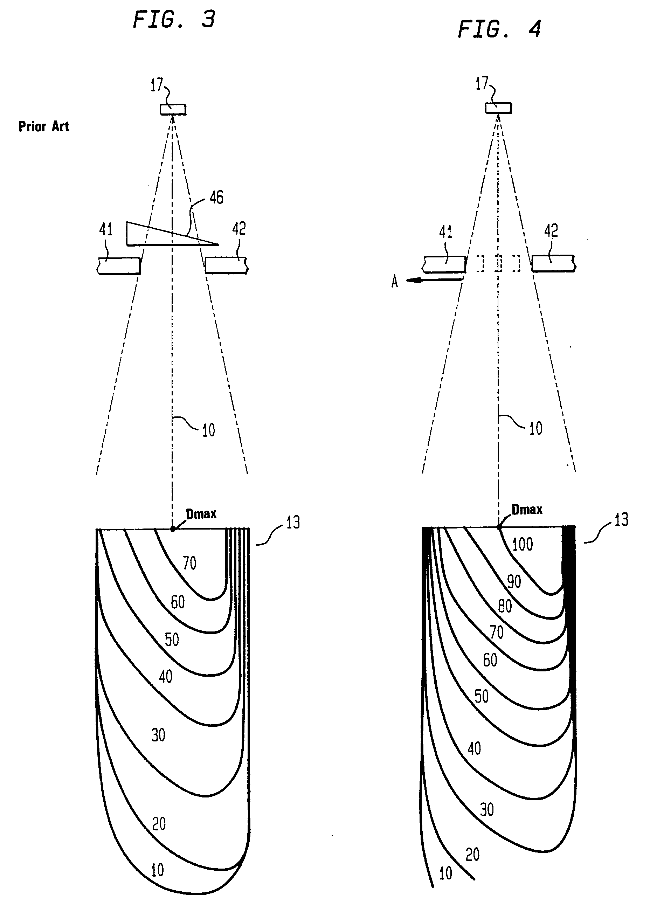

Figure 3 shows isodose curves for a conventional wedge filter 46 in the path of the radiation beam emitted from radiation source 17 to object 13. The radiation beam is shaped on the one hand by the wedge filter and on the other hand by aperture plates 41 and 42. Due to the absorbing material of wedge filter 46, the isodose curve in the center 10 of the beam on object 13 has a maximum value of Dmax, which is the maximum value at a spot in center 10 of the beam on the surface of object 13 without wedge filter 46. In the illustrated example, Dmax is roughly 72%. The wedge factor defined as the ratio of doses with and without wedge filter 46 is thus, in this case 0.72.

In a first embodiment of the present invention, a wedge-shaped absorber is simulated. Figure 4 shows isodose curves in a radiation treatment device according to the invention. Instead of including a wedge-shaped absorber in the path of the radiation beam, the filter function is performed by changing the radiation output of the radiation beam and by simultaneously moving at least one plate 41 and keeping the other plates of plate arrangement 4 stationary. A radiation treatment device having such a filter arrangement is disclosed in U.S. Pat. No. 5,148,032. Although this U.S. Patent describes the possibility of moving any plate, in the following, the invention is described in connection with only one plate being moved and the other plates being kept stationary. This is for the sake of simplicity only. The invention may be used for multiple moving plates as well.

When in Figure 4 plate 41 moves in the direction of arrow A toward plate 42 and at the same time the radiation output is changed according to a desired wedge angle, by adjusting the speed of plate 41 and/or correspondingly, the value of the isodose curve through the center of the beam on the surface of object 13 equals Dmax = 100%. Thus, by using a wedge function instead of a wedge-shaped absorber an efficiency factor of "1" or 100% can be established; in other words, the dose delivered at that point is 100% of the prescribed dose, although the same relative isodose profiles are maintained. That means that the therapist does not have to take into account a wedge factor when defining the treatment, although wedge shaped isodose curves are established.

Figure 2 shows those portions of treatment unit 100 which are necessary to carry out the invention. Treatment unit 100 comprises central processing unit 18 and which is programmed by the therapist according to the instructions of the oncologist so that the radiation treatment device carries out the prescribed radiation treatment. Through keyboard 19 the prescribed delivery of the radiation treatment is input.

Central processing unit 18 is connected, on the one hand, with the input device, such as the keyboard 19, for inputting the prescribed delivery of the radiation treatment and, on the other hand, with a dose control unit 61 that generates the desired values of radiation for the controlling trigger system 3. Trigger system 3 then adapts the pulse repetition frequency or other parameters in a corresponding, conventional manner. The ability to change the radiation output is generally known and it is particularly advantageous to use a digital dosimetry system because then it can easily be controlled by the digital output of central processing unit 18.

Central processing unit 18 includes control unit 76 which controls the execution of the program and which supplies position signals P for controlling the opening of plate arrangement 4 and nominal dose signals D (corresponding to the plate position that would be demanded using prior art methods, that is, without regard to output factor compensation) for adjusting the radiation output at the output of radiation source 17. A memory 77 is also provided in or is connected to the central processing unit 18 for supplying correction signals C, which the processing unit uses to adjust the radiation output dependent on the position signals P supplied from position sensors 44 and 45 in order to achieve the predetermined constant output factor.

The preferred arrangement of the memory unit is that, for each plate position (field size), it has stored a corresponding wedge correction signal C. The memory thus stores a table of wedge correction factors. If more than one set of movable plates is included in the system, then the table will be correspondingly multi-dimensional, and arranged using any known data structure, so that a wedge correction factor is available for any combination of plate positions.

Control unit 76 and memory 77 apply the nominal dose and wedge correction signals D and C, respectively, to a combination circuit 78, which combines the values to generate set signals S. The set signals S are in turn applied to the dose control unit 61, which sets the radiation output.

The combination circuit 78 will depend on the form in which the wedge correction signals are generated and stored. Assume that the wedge correction signals C are stored as additive offsets to the set radiation output. In this case, the combination circuit will be an adder which adds the wedge correction signals C to nominal dose signals D. This is the preferred embodiment, since it is simplest. If, however, the wedge correction factors are multipliers, for example, an increase in radiation output from a sensed value of 72% would require a multiplicative correction signal of about 139%. Instead of storing actual values of the wedge correction signals C, it is also possible to store the parameters of a wedge correction function for the various field sizes. The processing unit would then evaluate the wedge correction function for each current field size using the parameters stored in the memory, and would then generate the wedge correction signals (additive or multiplicative) itself.

The wedge correction signals are determined before actual treatment of a patient in one or more calibration runs. To determine relative wedge correction values, a reference surface is irradiated with a known reference plate position, and the radiation output over the surface is sensed by a conventional sensing device, which generates radiation output signals. These output signals are then applied to processing unit 18. In particular, the radiation output at a reference point (for example, at the center of the beam) is sensed. The reference surface need not lie in the patient plane, although if it does, the calibration will typically be easier and more accurate.

The plates are then moved to a new opening position, the radiation output is sensed and the needed amount of adjustment is determined to create the proper isodose profile for that position. This process is continued until correction values are stored for the reference surface over the entire range of motion of the plates. If more than one set of movable plates is included, then correction values will be sensed and stored for each combination of plate positions; the number of combinations will depend on the desired or required resolution.

The correction values indicate by how much the radiation output (for example, dose rate) is to be changed (via the wedge correction signals) such that the delivered dose distribution is equal to the desired dose distribution, that is, the isodose profiles are generated corresponding to what they would be if the radiation output were held constant and a physical wedge were included in the beam path. During actual treatment, for each plate position, the processing unit adjusts the radiation output to correspond to what is needed to generate the correct isodose profile. Since no actual physical wedge is included, however, and the system is calibrated for 100% output at the reference point, the therapist need not perform any calculations to adjust for a wedge factor. If additive offsets are chosen for the wedge correction factors, then the difference between the sensed output values and the desired output value is stored. If multiplicative correction factors are chosen, then ratios are stored. Alternatively, any known function approximation method may be used to generate the parameters of an approximating function of the additive or multiplicative wedge correction factors required.

A "course" of radiation treatment may, and often does, have more than one field, and may run over several different sessions. In some cases, hundreds of different (and, in some cases, fixed) sequential fields with different wedges are used during a course, for example, to provide proper irradiation of a field that has a complicated geometry or prescribed dose profile, to lessen discomfort to the patient, or to adjust the field as a tumor shrinks during treatment. The invention therefore also comprises an optional verification and recording or "auto set-up" system 102 (see Figure 2), which stores and downloads to the radiation system (via the CPU 18 or directly into the memory) the parameters, for example, of the geometry, of the various fields of the course of treatment, and/or the tables of wedge correction factors that were derived during earlier calibration runs for the various fields.

In a second embodiment of the present invention, collimator leaves and/or a wedge-shaped absorber are simulated. As is known in the art, a collimator containing multiple leaves (e.g., 60) can be used in place of or in addition to plates 41 and 42. When a collimator is used, the leaves are positioned such that they delimit the radiation beam to produce a pattern which closely matches the parameters of the field on the object to be irradiated. While the collimator is a helpful tool, it is expensive. A physical wedge also adds expense to a radiation treatment device. Some customers can only afford a less expensive radiation treatment device which provides less accuracy. This decrease in accuracy is acceptable when the alternative is no radiation treatment. The present embodiment, therefore, is provided without a multi-leaf collimator and without a physical wedge; instead, the multi-leaf collimator and the physical wedge are simulated.

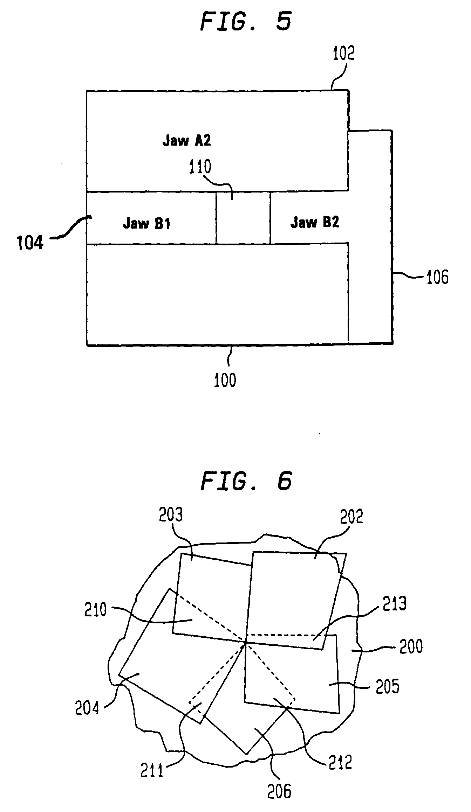

Figure 5 illustrates four jaws located in a radiation treatment device between the radiation source and the object. These jaws 100, 102, 104 and 106 contain no additional plates or leaves, but are capable of blocking radiation from the radiation source. In the preferred embodiment, jaws 100 and 104 are positioned in a predetermined location (e.g., 10cm), and jaws 102 and 106 are later modulated during the radiation treatment session. This modulation changes the dimensions of beam opening 110. The radiation beam travels from the radiation source and through opening 110 before reaching the object to be treated. Jaws 100, 102, 104, 106 are composed of a radiation blocking material. Therefore, when the radiation beam passes through opening 110, it is delimited to the shape of opening 110. Jaws 100 and 104 are modulated during the treatment session such that opening 110 has desired parameters and the radiation beam is delimited accordingly.

In addition to changing the shape of opening 110, the radiation treatment device can also be rotated, preferably, at least 360 degrees during the radiation treatment. This rotation changes the position of the radiation source which emits the radiation beam. Therefore, the radiation beam can be administered from various positions around the field to be irradiation. For example, the radiation source can be located directly above the field at top center, or 10 degrees right of top center, etc. Figure 1 illustrates how gantry 6 rotates around axis 8 such that the position of the source of radiation can be changed.

Also, in addition to changing the position of the radiation source, the radiation output of the radiation beam can be changed. To simulate a multi-leaf collimator and a physical wedge, the field to be irradiated is treated while changing the radiation output of the radiation beam, while rotating the radiation treatment device (and thereby rotating the radiation beam source) and while simultaneously moving jaws 102 and 106.

Figure 6 illustrates beam patterns created by the second embodiment of the present invention. The field to be irradiated 200 is treated with multiple radiation beam patterns 202-206. For example, radiation beam pattern 202 results from (1) a specific radiation output from the radiation beam, (2) the position of jaws 100, 102, 104 and 106 (e.g., 5cm and 7.5cm), and (3) the position of the radiation treatment device (e.g., 60 degrees from top center). The beam pattern can result in a wedge-like distribution of radiation, and it can provide coverage similar to that resulting from use of a multi-leaf collimator and/or a physical wedge. For example, overlapping areas 210-213 have a higher concentration of radiation then other areas within radiation beam patterns 202-206. Beam patterns are created and overlaid on top of one another such that a desired treatment is obtained.

Again, as in the first embodiment, correction signals are supplied by central processing unit 18. These correction signals include (1) position signals P for controlling the position of jaws 100, 102, 104, 106, (2) nominal dose signals D for adjusting the radiation output at the radiation source, and (3) rotation position signals R for controlling the rotational position of the radiation source. In this embodiment, central processing unit 18 performs complex calculations to provide the beam patterns which will cover the field to be irradiated in the most complete manner possible. The software program used for these complex calculations along with the calculated beam patterns are stored in memory 77. These stored beam patterns are later used for the radiation treatment. In the preferred embodiment, the complex calculations begin with the inputted dimensions of the field to be irradiated. This is preferably done in three dimensions such that depth is also taken into consideration. Beam patterns, based on signals P, D and R, are then used to "fill up" the inputted dimensions (as shown in Figure 6). Again, this is done by overlaying multiple beam patterns. After the beam patterns are determined, the associated corrective signals for (1) the position of the jaws, (2) the radiation dose level, and (3) the position of the radiation source are stored in memory 77. Thus, a set of corrective signals for each beam pattern is utilized. These corrective signals are used in the same manner as described in the first embodiment of the present invention.

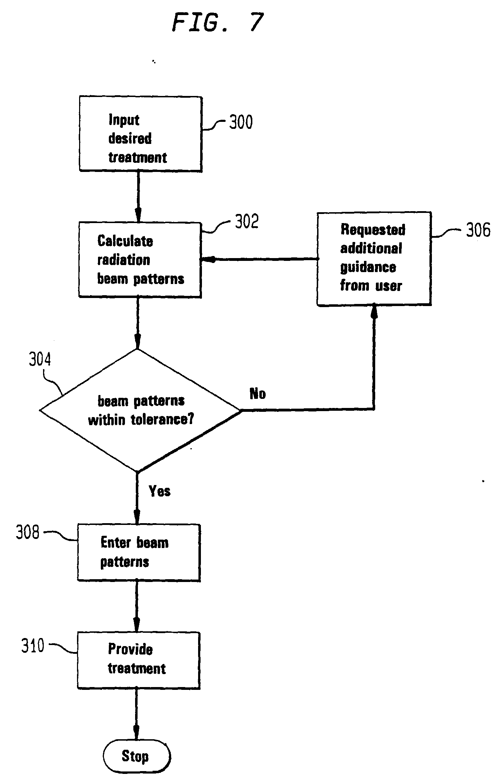

Figure 7 provides a process flowchart for a simple software program which can be used to quickly calculate beam patterns for a desired wedge factor. Preferably, central processing unit 18 (see Figure 1) contains this software program. At step 300 a user inputs a desired treatment for the patient. This desired treatment includes the parameters of the field to be irradiated in three dimensions. At step 302, the software uses available information to calculate corresponding beam patterns. The available information includes (1) the field parameters inputted by the user, (2) the available positions of jaws 102 and 106, (3) the minimum and maximum radiation output from the radiation beam, and (4) the available positions of the radiation treatment device. The beam patterns "fill up" the inputted parameters. At step 304, the software checks the calculated beam patterns. The available information may provide for inaccurate coverage of the field to be irradiated. If these beam patterns are not within a predetermined tolerance, the software requests additional information at step 306. For example, if the system can only achieve a 95% or 110% treatment with the available information, and these treatment percentages are outside the predetermined tolerance, then the user will be asked to change the desired treatment or pick between the available treatments (e.g., 95% and 110%) at step 306. This continues until a desired treatment falls within the predetermined tolerance or is selected by a user. When the calculated beam patterns are within the desired tolerance or selected by a user, the beam patterns are stored in the memory at step 308. Treatment is then provided at step 310.

Various radiation treatment techniques can be utilized with the second embodiment of the present invention. In addition to the random pattern technique shown in Figure 6, a sweeping technique and a quadrant technique can be used. These additional techniques are desirable because they provide move control over the disposition of radiation. For example, sometimes an object has a sensitive area near the field to be treated which can be greatly damaged by extensive radiation exposure (e.g., spinal cord area). Increased control over the disposition of radiation can allow for thorough treatment without extensive exposure to the sensitive area. Thus, the sweeping technique and the quadrant technique create beam patterns which can provide better treatment of the field to be irradiated.

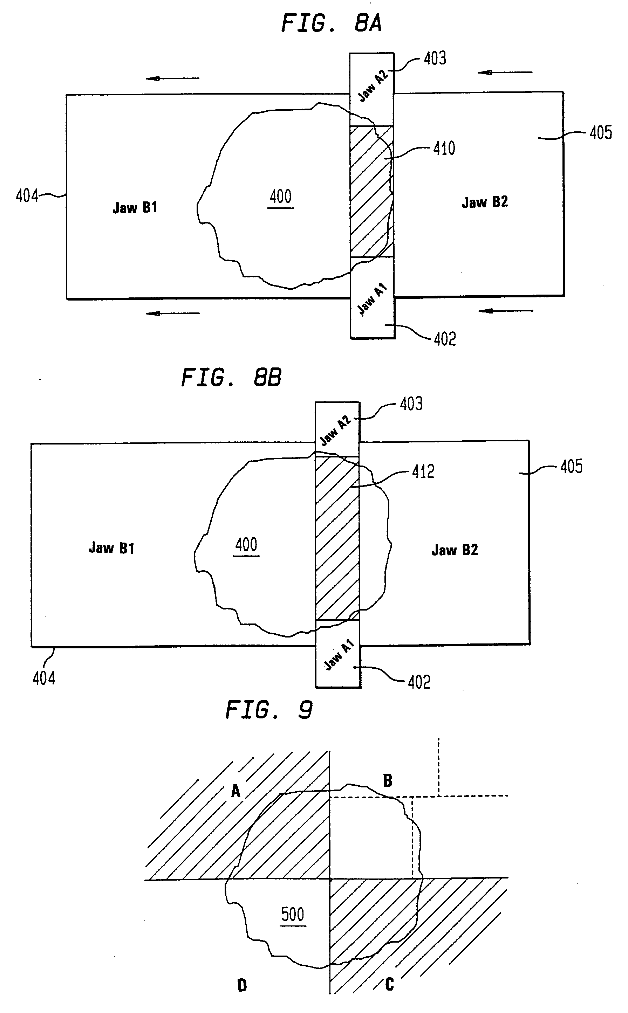

Figures 8A and 8B illustrate beam patterns created by a sweeping technique. This sweeping technique is used to irradiate field 400 which is located on the object to be treated. As shown in Figure 8A, Jaws 402-405 define slotted opening 410. Slotted opening 410 can be, for example, .5cm by 10cm. The radiation is provided to a portion of field 400 through slotted opening 410. Jaws 402-405 are then repositioned as shown in Figure 8B. This repositioning is done by moving all of jaws 402-405 to the left. Jaws 402-405 can move (or sweep) across field 400 at the same velocity to create a second slotted opening which has the same dimensions as first slotting opening 410, or jaws 402-405 can move at different velocities to create slotted opening 412 which has different dimensions from slotted opening 410. After jaws 402-405 are repositioned to create slotted opening 412 (shown in Figure 8B), radiation is provided to another portion of field 400 through slotted opening 410. This is continued until all of field 400 is treated. In another arrangement, radiation is continually provided through the slotted openings defined by jaws 402-405 while jaws 402-405 are sweeping across field 400. In both arrangements, jaws 402-405 sweep across field 400 until the field is irradiated. Overlapping can occur between different slotted openings (e.g., between slotted openings 410 and 412). This overlapping in radiation treatment is taken into consideration during the treatment planning process. Complex calculation are done to determine the position of each slotted opening and to determine the desired radiation deposition dose for each defined slotted opening.

Figure 9 illustrates a beam pattern created by a quadrant technique. Field 500 is the area on the object to be treated. In this arrangement, field 500 is divided up into portions, and treatment is provided to each portion separately. Figure 9 provides an example of dividing up field 500 into quadrants A, B, C and D. Each quadrant is a portion. In the preferred embodiment, two of the jaws remain stationary, while the other two jaws are modulated to cover the portion of field 500 to be treated. For example, quadrants A and C can be covered by immobile jaws which define the left hand side and the bottom of quadrant B. Mobile jaws can then be utilized to define the right hand side and top of quadrant B. After quadrant B is defined with the jaws and treated with radiation, the jaws are repositioned to define another quadrant. This is continued until all quadrants A, B, C and D are treated with radiation. The axises can then be slightly rotated to define new quadrants, and treatment can be repeated for better coverage of field 500. Again, complex calculations are used to determine how much radiation should be deposited in each defined quadrant/portion.

In yet another arrangement, both the sweeping technique and the quadrant technique can be used together to provide better coverage of the field to be irradiated. Also, as described above, the position of the radiation source can be rotated and the radiation output from the radiation beam can be changed. Each or both of these parameters, in addition to the sweeping and/or quadrant techniques, can be used to better simulate a multileaf collimator and a physical wedge.

## Claims

1. A method for adjusting radiation output delivered to an object from a radiation source, the radiation source being rotatable to available positions, comprising the following steps:
defining a field on the object for irradiation, the field being defined by field parameters;
generating a radiation beam having a variable radiation output and a substantially lossless beam path from a radiation source to the object;
defining an opening between the radiation source and the object, the opening having multiple available parameters, the opening capable of delimiting the radiation beam to any one of the multiple available parameters;
calculating beam patterns based on (i) the field parameters, (ii) the available positions of the radiation source, (iii) a minimum and a maximum amount of radiation available from the variable radiation output and (iv) the multiple available parameters for the opening, wherein the beam patterns each include a position for the radiation source, an amount of radiation from the radiation output and parameters for the opening; and
treating the field on the object with radiation by varying the position of the radiation source, the amount of radiation from the radiation output and the parameters of the opening according to the calculated beam patterns.

2. The method for adjusting radiation output delivered to an object from a radiation source of claim 1, further comprising the step of calculating corrective signals based on (I) position signals for controlling the parameter of the opening, (ii) dose signals for adjusting the radiation output and (iii) rotation position signals for controlling the position of the radiation source.

3. The method for adjusting radiation output delivered to an object from a radiation source of claim 1, wherein the opening is defined by at least one jaw, the jaw being capable of blocking radiation from the radiation source.

4. The method for adjusting radiation output delivered to an object from a radiation source of claim 3, wherein four jaws are used to define the opening, and wherein two of the four jaws are modulated during the treatment of the field.

5. The method for adjusting radiation output delivered to an object from a radiation source of claim 1, further comprising the step of storing the beam patterns in a memory.

6. The method for adjusting radiation output delivered to an object from a radiation source of claim 1, whereby a wedge-like distribution of radiation is obtained.

7. The method for adjusting radiation output delivered to an object from a radiation source of claim 1, wherein the field parameters are in three dimensions, and wherein the calculating of the beam patterns is done for three dimensional treatment.

8. The method for adjusting radiation output delivered to an object from a radiation source of claim 1, wherein the multiple available parameters for the opening provide for beam patterns created by at least one of a sweeping technique and a quadrant technique.

9. A method for adjusting radiation output delivered to an object from a radiation source, the radiation source being rotatable to available positions, the radiation source being capable of generating a radiation beam, the radiation beam having variable radiation output, the method comprising the steps of:
inputting parameters of a field on the object for irradiation;
calculating beam patterns with available information, the beam patterns corresponding to the inputted parameters of the field, and the available information including (i) the inputted parameters, (ii) the available positions of the radiation source, (iii) a minimum and a maximum amount of radiation available from the variable radiation output and (iv) available parameters defined by an opening, the opening being located between the radiation source and the object such that the radiation beam is delimited to the parameters of the opening, wherein the calculated beam patterns fill up the inputted parameters of the field; and
treating the field on the object with radiation.

10. The method for adjusting radiation output delivered to an object from a radiation source of claim 9, further comprising the steps of:
checking the calculated beam patterns; and
requesting additional information when the calculated beam patterns fail to meet tolerances for filling up the inputted paramenters of the field.

11. The method for adjusting radiation output delivered to an object from a radiation source of claim 9, further comprising the step of storing the calculated beam patterns in a memory.

12. The method for adjusting radiation output delivered to an object from a radiation source of claim 9, further comprising the step of calculating corrective signals based on (I) position signals for controlling the parameter of the opening, (ii) dose signals for adjusting the radiation output and (iii) rotation position signals for controlling the position of the radiation source.

13. The method for adjusting radiation output delivered to an object from a radiation source of claim 9, wherein the opening is defined by at least one jaw, the jaw being capable of blocking radiation from the radiation source.

14. A system for adjusting radiation output delivered to a field to be irradiated on an object, the radiation output originating from a radiation source, comprising:
a radiation source generating a radiation beam having a variable radiation output;
beam-shielding means for delimiting the output radiation beam to predetermined parameters;
a dose controller for varying an amount of the radiation output from the radiation source;
a position controller for varying the rotational position of the radiation source; and
processing means for generating beam patterns, the beam patterns providing data for filling up a field on the object with radiation, and the beam patterns being based on capabilities of the beam-shielding means, the dose controller and the position controller;
wherein the beam patterns are used to treat the field on the object with radiation by varying (I) the rotational position of the radiation source with the position controller, (ii) the amount of radiation output from the radiation source with the dose controller and (iii) the position of the delimiting beam-shielding means.

15. The system for adjusting radiation output delivered to a field to be irradiated on an object of claim 14, wherein corrective signals are calculated, the corrective signals being based on (I) position signals for controlling the position of the beam-shielding means, (ii) dose signals for adjusting the radiation output and (iii) rotation position signals for controlling the position of the radiation source.

16. The system for adjusting radiation output delivered to a field to be irradiated on an object of claim 14, further comprising a memory for storing the beam patterns.

17. The system for adjusting radiation output delivered to a field to be irradiated on an object of claim 14, wherein the beam-shielding means are jaws.

18. The system for adjusting radiation output delivered to a field to be irradiated on an object of claim 17, wherein a portion of the jaws are stationary and a portion of the jaws are modulated during the radiation treatment.

19. The system for adjusting radiation output delivered to a field to be irradiated on an object of claim 14, whereby a wedge-like distribution of radiation is obtained.

20. The system for adjusting radiation output delivered to a field to be irradiated on an object of claim 14, wherein the field on the object is three dimensions, and wherein the calculating of the beam patterns is done for three dimensional treatment.

21. The system for adjusting radiation output delivered to a field to be irradiated on an object of claim 14, wherein the beam patterns are created by at least one of a sweeping technique and a quadrant technique.

22. A method for adjusting radiation output delivered to an object from a radiation source, the radiation source being rotatable to available positions, the radiation source being capable of generating a radiation beam, the radiation beam having variable radiation output, the method comprising the steps of:
inputting parameters of a field on the object for irradiation;
calculating beam patterns with available information, the beam patterns corresponding to the inputted parameters of the field, and the beam patterns created by at least one of a sweeping technique and a quadrant technique, the opening being located between the radiation source and the object such that the radiation beam is delimited to the parameters of the opening, wherein the calculated beam patterns fill up the inputted parameters of the field; and
treating the field on the object with radiation.

23. The method for adjusting radiation output delivered to an object from a radiation source of claim 22, further comprising the step of storing the calculated beam patterns in a memory.

24. The method for adjusting radiation output delivered to an object from a radiation source of claim 22, further comprising the step of calculating corrective signals based on (I) position signals for controlling the parameter of the opening and (ii) dose signals for adjusting the radiation output.
